(19) Europäisches Patentamt
European Patent Office
Office européen des brevets

(11) EP 0 589 069 B1

(12) EUROPEAN PATENT SPECIFICATION

(45) Date of publication and mention of the grant of the patent: **29.01.1997 Bulletin 1997/05**

(51) Int Cl.[6]: **C07C 43/23**, C09K 15/08, C11B 5/00, A61K 7/00, A61K 31/085

(21) Application number: **92116002.4**

(22) Date of filing: **18.09.1992**

(54) **Hydroquinone derivatives and antioxidants and tumor inhibitors containing them**

Hydrochinonderivate und diese enthaltende Antioxydantien sowie Tumorinhibitoren

Dérivés d'hydroquinone et des antioxydants aussi bien que des inhibiteurs de tumeurs les contenant

(84) Designated Contracting States:
**DE FR GB**

(43) Date of publication of application:
**30.03.1994 Bulletin 1994/13**

(73) Proprietor: **NIPPON HYPOX LABORATORIES INCORPORATED**
**Tokyo 192-03 (JP)**

(72) Inventors:
- **Satoh, Toshio**
  **Tokushima-shi, Tokushima-ken (JP)**
- **Matsumoto, Hitoshi**
  **Tokushima-shi, Tokushima-ken (JP)**
- **Niiro, Yasunori**
  **Tokushima-shi, Tokushima-ken (JP)**

(74) Representative: **Türk, Gille, Hrabal, Leifert**
**Brucknerstrasse 20**
**40593 Düsseldorf (DE)**

(56) References cited:

| | |
|---|---|
| **DD-A- 287 482** | **GB-A- 621 519** |
| **GB-A- 1 441 735** | **US-A- 2 235 884** |
| **US-A- 2 666 708** | **US-A- 2 679 459** |
| **US-A- 2 704 724** | **US-A- 2 908 718** |
| **US-A- 4 552 682** | |

- **CHEMICAL ABSTRACTS, vol. 76, no. 5, 31 January 1972, Columbus, Ohio, US; abstract no. 23828j, T. YAMANO et al, "Preparation of Japanese sake", page 261, column 2; & JP-A-7 109 236**
- **CHEMICAL ABSTRACTS, 9th Collective Index, Chemical Substances, American Chem. Soc., p. 27808CS, col. 1: phenol, 4-(heptyloxy), p. 27967CS, col. 2: phenol, 4-(nonyloxy)**
- **CHEMICAL ABSTRACTS, 11th Collective Index, Chemical Ssubstances, American Chem. Soc., p. 48794CS, col. 3: phenol, 4-(undecyloxy)**
- **CHEMICAL ABSTRACTS, vol. 68, no. 1, 1 January 1968, Columbus, Ohio, US; abstract no. 2118x, J. POSPISIL et al, "Antioxidants", page 186, column 2; & CS-A-119834**
- **CHEMICAL ABSTRACTS, vol. 96, no. 15, 12 April 1982, Columbus, Ohio, US; abstract no. 121805g, E. BASALKEVICH et al, "Reaction of isoprenoid carbinols and polyfunctional phenols with titanium tetrachloride", page 611, column 1**
- **BERICHTE DER DEUTSCHEN CHEMISCHEN GESELLSCHAFT, vol. 73, no. 9, 4 September 1940, Weinheim, DE, pages 995-1001, W. JOHN et al, "Über die Synthese und Struktur der Pseudocumohydrochinon-mono-alkyläther"**
- **CHEMICAL ABSTRACTS, vol. 32, no. 20, 20 October 1938, Columbus, Ohio, US; F. V. WERDER et al, "Synthetic compounds with vitamin E activity", column 7972**
- **CHEMICAL ABSTRACTS, vol. 32, no. 12, 20 October 1938, Columbus, Ohio, US; W. JOHN et al, "Antisterility factors (vitamin E). III. Hydriodic acid splitting of tocopherols", column 4601**
- **CHEMICAL ABSTRACTS, vol. 104, no. 13, 31 March 1986, Columbus, Ohio, US; abstract no. 109228x, K. TANIGUCHI, "Hydroquinone monoethers", page 685, column 2; & JP-A-60 215 643**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 589 069 B1

## Description

The present invention relates to novel hydroquinone derivatives, and an antioxidant and a tumor incidence inhibitor each containing hydroquinone derivatives as active ingredient.

### Background of the Invention

Foods, feeds and cosmetics contain antioxidants (oxidation preventers) as required in order to prevent color fading or discoloration, a change in the aroma and formation of peroxide during the preservation.

Examples of the antioxidants for the above use include the following.

(1) For foods and feeds

Butylhydroxytoluene (hereinafter referred to as BHT), dl-α-tocopherol, nordihydroguaiaretic acid, butylhydroxyanisole (hereinafter referred to as BHA) and propyl gallate.

(2) For cosmetics

BHT, dl-α-tocopherol, BHT and gallic acid.

Being excellent in antioxidant activity and less expensive, BHT is particularly preferentially used as a fat-soluble antioxidant for foods and as a fat-soluble antioxidant for cosmetics.

Meanwhile, with recent eating habits diversified, not only a variety of biological sources are put on the market, but also a variety of processed foods having new ingredients are being developed. With an aesthetical sense uplifted in the field of cosmetics in recent years, a variety of cosmetics having new ingredients are as well being developed.

An immunological study has revealed that the occurrence of human cancer is closely related to a daily life, particularly to eating habits and smoking. In recent years, it has been found that mutagens are developed by heating foods, and that most of these mutagens are nitrogen-containing aromatic heterocyclic compounds having an amino group (heterocyclic amine) (Sugiura T, et al., Perspectives on the Relative Risks, p.31, Mercell Dekker Inc, New York, 1989).

Above all, it has been revealed that 2-amino-6-methyldipyrido[1,2-a:3',2'-d]imidazole (Glu-P-1) formed by heating L-glutamic acid not only exhibits high mutagenic activity to Salmonellae but also exhibits carcinogenic activity to laboratory animals. An experiment with rats has shown that Glu-P-1 has a high rate of incidence of cancer particularly on the liver, large intestine, small intestine and gland of external acoustic meatus (Tkayamz S, et al., Gann, 75, 207, 1984).

US-A-2,679,459 discloses alkoxyphenols having 2 additional alkyl substituents which can be used for stabilization of unsaturated gasoline or stabilization of edible fats and oils.

Chem. Abs. 1982, 96(15) 121805g, Berichte der Deutschen Chemischen Gesellschaft, 1940, 73(9), 995-1001, Chem. Abs., 1938, 32(20), col. 7972, Chem. Abs., 1938, 32(12), col. 4601 and US-A-2,235,884 disclose alkoxyphenols having 3 additional substituents, wherein the alkoxy group is a $C_{2-19}$ alkoxy group. Chem. Abs. 1938, 32(20), col. 7972 and Chem. Abs. 1938, 32(12), col. 4601 additionally disclose alkoxyphenols having 4 additional methyl substituents, wherein the alkoxy residue is a $C_{4-16}$ alkoxy group. Some of these compounds are reported to possess a vitamin E-activity.

It is considered that the risk of carcinogenesis on humans can be avoided or decreased if the mutagenic activity and carcinogenic activity of heterocyclic amine can be inhibited. In particular, the transition of chronic hepatitis and cirrhosis to cancer of the liver is highly liable to occur, and it is a large clinical problem. Not an anti-cancer drug which directly attacks cancer cells, but a compound having a tumor incidence inhibiting activity is considered to work effectively on patients having such diseases.

### Summary of the Invention

It is an object of the present invention to provide a novel hydroquinone derivative having antioxidant activity, and use of hydroquinone derivatives as antioxidant, for the preparation of a medicament for prevention and therapy of functional disorders in organs, for the preparation of a medicament for eliminating tissue disorder-inducing factors such as active oxygen species and active organic radical species or the preparation of a medicament for inhibition of tumor incidence.

The above object of the present invention is achieved firstly by a hydroquinone derivative of the formula (IIa).

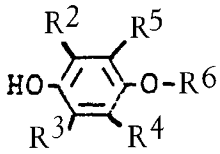

... (IIa)

wherein $R^2$ to $R^6$ are defined as in claim 1.

The above object of the present invention is further achieved by use of a hydroquinone as antioxidant wherein said hydroquinone is represented by the formula (IIb),

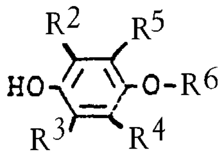

... (IIb)

wherein each of $R^2$ to $R^6$ is defined as in claim 3.

The above object of the present invention is further achieved by use of the above compound of formula (IIb) and (IIc) for the preparation of various medicaments.

The compound of formula (IIc) is a hydroquinone derivative of the formula :

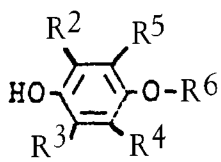

wherein $R^2$ to $R^6$ are defined as in claim 7.

Brief Description of Drawing

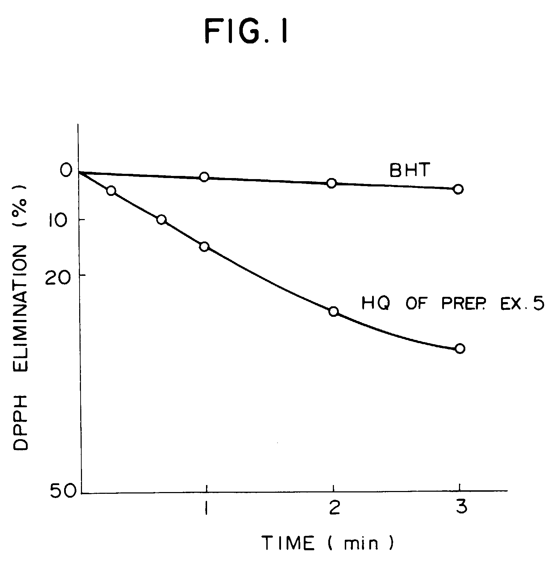

Fig. 1 is a graph showing test results on DPPH elimination ability.

Detailed Description of the Invention

First, the hydroquinone derivative A of the present invention will be explained below.

The hydroquinone derivative A is a compound having the formula (IIa) as described above.

The hydroquinone derivative obtained as above has antioxidant activity sufficient to be utilized as an antioxidant.

The hydroquinone derivatives of formulas (IIa), (IIb) and (IIc) of the present invention will be explained hereinafter.

The hydroquinone derivatives (IIa) to (IIc) are compounds having the formulas as described above. In the formulas (IIa) to (IIc) each of $R^2$ to $R^5$ is independently $C_{1-4}$ alkyl group and $R^6$ is an alkyl group having a carbon chain as defined in claim 1, 3 and 7. The $C_{1-4}$ alkyl group as $R^2$ to $R^5$ includes methyl, ethyl, propyl and butyl. The alkyl group having a carbon chain of at least 2 carbon atoms as $R^6$ includes ethyl, n-propyl, n-butyl, n-pentyl, n-hexyl, n-heptyl, n-octyl. n-nonyl, n-decyl, n-undecyl, n-dodecyl, n-tridecyl, n-tetradecyl, n-pentadecyl, n-hexadecyl, n-heptadecyl and n-octadecyl. In case of compound (IIa) the chain of $R^6$ includes n-octyl. In case of compound (IIb) the chain of $R^6$ includes n-butyl, n-pentyl, n-hexyl, n-heptyl, and n-octyl.

The hydroquinone derivative of the formulas (IIc), (IIa) and (IIb) can be obtained, for example, from a known hydroquinone derivative of the formula (IV) as a starting material,

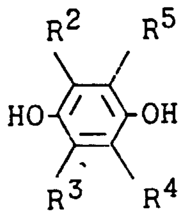

... (IV)

wherein $R^2$ to $R^5$ are as defined in the formulas (IIc), (IIa) and (IIb) by the following method (a).

(a) When it is intended to obtain, for example, a hydroquinone derivative wherein one hydroxyl group which is under less steric hindrance than the other hydroxyl group is etherified as shown in the hydroquinone derivative of the following formula,

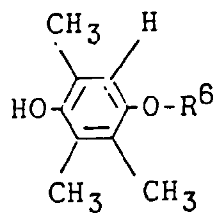

the hydroquinone derivative of the formula (IV) is allowed to react with an alcohol of the formula,

$$R^6\text{-OH}$$

wherein $R^6$ is the same as $R^6$ in the formula (IIc), (IIa) or (IIb), in the presence of a heteropoly-acid such as phosphomolybdic acid, phosphotungstic acid, silicomolybdic acid or silicotungstic acid.

The hydroquinone derivative obtained by the above method has antioxidant activity sufficient to be utilized as an antioxidant.

The use as an antioxidant of the present invention will be explained hereinafter.

The antioxidant contains the hydroquinone derivative of the formula (IIb) as an active ingredient as described previously.

As is clear in the formula (IIb), the antioxidant of the present invention includes the hydroquinone derivative (IIa) and known hydroquinone derivatives which have been found for the first time to have excellent antioxidant activity by the study of the present inventors.

The antioxidant of the present invention may contain conventional carriers such as starch, crystalline cellulose, acacia (Arabic gum), lactose, sucrose, glucose, glycerol, propylene glycol, ethanol, etc.

The antioxidant of the present invention is useful as an antioxidant for use in foods, feeds and cosmetics due to its excellent antioxidant activity and low toxicity. Further, the hydroquinone (IIb) according to the present invention can be used for the preparation of a medicament for the prevention and therapy of functional disorder of organs, since it can eliminate tissue disorder-inducing factors such as active oxygen species and active organic radical species. The compound (IIc) can further be used for the preparation of a medicament for the inhibition of tumor incidence.

The tumor incidence inhibitor of the present invention has the activity to inhibit the mutagenic activity and carcinogenic activity of heterocyclic amine. Further, this tumor incidence inhibitor exhibits no activity to destroy normal cells. and its toxicity test data has shown its high safety.

The tumor incidence inhibitor contains the hydroquinone derivative of the formula (IIc) as an active ingredient as described previously.

As is clear in the formula (IIc), the tumor incidence inhibitor of the present invention includes the hydroquinone derivatives (IIa) and (IIb) and other known hydroquinone derivatives which have been found for the first time to have excellent tumor incidence inhibiting activity by the study of the present inventors.

The tumor incidence inhibitor of the present invention may contain conventional carriers such as starch, crystalline cellulose, acacia (Arabic gum), lactose, sucrose, glucose, glycerol, propylene glycol, ethanol, etc.

Examples

The present invention will be explained hereinafter by reference to Examples.

(Reference Example 1)

[Preparation of 1-octylhydroquinone,

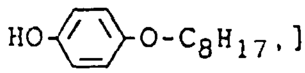

0.7 Gram of phosphomolybdic acid ($P_2O_5 \cdot 24MoO_3 \cdot xH_2O$) was added to a solution of 2.2 g (20 mmol) of hydroquinone in 40 ml of n-octyl alcohol. The resultant mixture was stirred, and then heated at 120°C for 6 hours. After the heating, 300 ml of each of water and EtOAc was added to the mixture, and the mixture was shaken.

Then, an organic layer was, recovered, dried over anhydrous $MgSO_4$ and concentrated under reduced pressure. The resultant residue was subjected to silica gel chromatography for elution with a mixed solution of hexane and EtOAc to give a crude product of 1-octylhydroquinone.

The above-obtained crude product was recrystallized from n-hexane to give 2.7 g of the intended, captioned compound (yield 60 %).

Table 1 shows the yield amount, yield, melting point and δ value in H-NMR with regard to the above-obtained 1-octylhydroquinone.

Preparation Example 1

[Preparation of 1-butyl-2,3,5-trimethylhydroquinone,

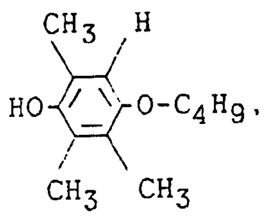

one of hydroquinone derivatives (IIc) or (IIb)]

The Reference Example 1 was repeated except that the hydroquinone was replaced with 2,3,5-trimethylhydroquinone and that the n-octyl alcohol was replaced with n-butyl alcohol to give 3.19 g of the intended, captioned compound (yield 73 %).

Table 1 shows the yield amount, yield, melting point and δ value in H-NMR with regard to the above-obtained 1-butyl-2,3,5-trimethylhydroquinone.

Preparation Examples 2 - 5

The Reference Example 1 was repeated except that the n-octyl alcohol was replaced with an alcohol of the formula, $R^6$-OH in which $R^6$ was as shown in Table 1, to give a hydroquinone derivative of the general formula II in which each of $R^2$, $R^3$ and $R^4$ was methyl, $R^5$ was hydrogen and $R^6$ was an alkyl shown in Table 1.

Table 1 shows the yield amount, yield, melting point and δ value in H-NMR with regard to each of the so-obtained hydroquinone derivatives B.

Reference Example 2

[Preparation of 1-ethyl-2,3;5-trimethylhydroquinone,

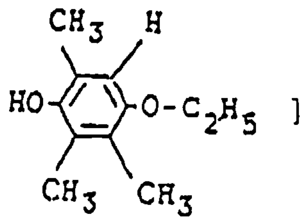

Preparation Example 1 was repeated except that n-octyl alcohol was replaced by ethyl alcohol to obtain the intended captioned compound (yield 73%).

Table 1 shows the melting point, and value in H-NMR of the obtained 1-ethyl-2,3,5-trimethylhydroquinone.

Table 1

| | Formula | $R^1$ or $R^6$ | Yield (percentage) | Melting point (°C) | δ-Value in H-NMR [CDC13] |
|---|---|---|---|---|---|
| Reference Example 1 | 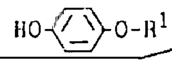 | $-C_8H_{17}$ | 2.6 (60 %) | 58.5-59 | 0.88 (3H,t,J=6.2 Hz), 1.31-1.96 (12H,m) 3.89 (2H,t,J=6.4 Hz), 6.76 (4H,s) |
| Example 1 | 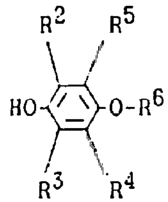 | $-C_4H_9$ | 3.19 (73 %) | 65.5-66.5 | 0.97 (3H,t,J=6.5Hz), 2.14 (3H,s) 1.30-1.90 (4H,m), 2.17 (3H,s), 2.21 (3H,s) 3.87 (2H,t,J=5.9Hz), 6.51 (H,s) |
| Example 2 | | $-C_6H_{13}$ | 3.17 (64 %) | 72.5-73 | 0.97 (3H,t,J=6.6Hz), 1.30-1.90 (8H,m) 2.14 (3H,s), 2.17 (3H,s), 2.21 (3H,s) 3.87 (2H,t,J=6.2Hz), 6.58 (H,s) |
| Example 3 | | $-C_8H_{17}$ | 2.99 (54 %) | 70-71 | 0.89 (3H,t,J=6.2 Hz), 1.10-1.76 (12H,m) 2.14 (3H,s), 2.17 (3H,s), 2.21 (3H,s) 3.86 (2H,t,J=6.2Hz), 6.52 (H,s) |
| Example 4 | ($R^2$=$R^3$=$R^4$ =$CH_3$, $R^5$=H) | $-C_{10}H_{21}$ | 2.57 (42 %) | 76-77 | 0.88 (3H,t,J=5.1 Hz), 1.05-1.94 (16H,m) 2.14 (3H,s), 2.17 (3H,s), 2.21 (3H,s) 3.86 (2H,t,J=6.4Hz), 6.51 (H,s) |
| Example 5 | | $-C_{12}H_{25}$ | 3.62 (55 %) | 81-83 | 0.88 (3H,t,J=6.4Hz), 1.26-1.97 (20H,m) 2.14 (3H,s), 2.17 (3H,s), 2.21 (3H,s) 3.86 (2H,t,J=6.2Hz), 6.51 (H,s) |
| Reference Example 2 | 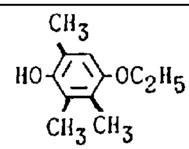 | - | (70 %) | 87-88 | 1.39 (3H,t), 2.14 (3H,s), 2.17 (3H,s), 2.21 (3H,s), 3.87 (2H,q), 4.24 (H,s), 6.58 (H,s) |

Antioxidant Activity Test Example 1

[Inhibition activity against hyperoxidation of lipid of rat liver microsome]

(1) Preparation of rat liver microsome suspension

A plurality of male SD rats (weighing 250 to 280 g) were prepared. Each rat was anesthetized with pentobarbital, and then subjected to celiotomy. A cannula was introduced into the portal vein of each rat, the abdominal large veins were sectioned, and 500 ml of cold 0.9 X NaCl was injected into the portal veins.

Livers were extracted from individual rats, and the livers were cut to pieces in 1.15 % KCl and homogenized with ice cooling. Then, 1.15 % KCl was further added to obtain a 30 % liver homogenized solution.

The above homogenized solution was centrifugally separated at 8.000 g (g: gravitation acceleration) for 10 minutes, and the resultant supernatant was recovered. The supernatant was centrifugally separated at 105,000 g (g: gravitational acceleration) for 1 hour, and 1.15 % KCl was added to the resultant residue such that the residue had a protein concentration of 10 mg/ml when measured for a protein amount by a Lowry method, thereby to obtain a liver microsome suspension.

The so-prepared suspension was stored at -20°C before a test described in the following (2), and it was used within 1 week after the preparation.

(2) Test on inhibition activity against hyperoxidation of lipid

As test compounds, the hydroquinone derivatives obtained in Reference Examples 1 and 2, Preparation Examples 1 to 5, a known hydroquinone derivative which was a compound of the formula (hereinafter referred to as HQ1)

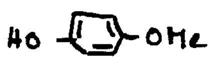

and BHT were respectively tested on their inhibition activities against hyperoxidation of lipid in the following manner.

First, 0.1 ml of the liver microsome suspension obtained in the above (1), 0.1 ml of NADPH (final concentration 2 mM), 0.1 ml of ADP (final concentration 10 mM) and 0.1 ml of a solution of one of the test compounds whose concentration was adjusted as shown in Table 2 with 10 % DMF were added to a tris-HCl buffer (167 mM KCl, 74 mM TRIS, pH 7.4), and the resultant mixture was warmed at 37°C for 5 minutes. Then, 0.1 ml of $FeCl_3$ (final concentration 0.1 mM) was added, and the mixture was warmed at 37°C for 20 minutes.

The resultant reaction mixture was cooled with ice. Then, 0.2 ml of 8.1 % SDS, 1.5 ml of an acetic acid buffer (prepared by adjusting 20 % acetic acid containing 0.27 mM HCl to pH 3.5 with 10N NaOH) and 1.5 ml of 0.8 X thiobarbituric acid were added to the cooled reaction mixture, and the resultant mixture was warmed over a boiling water bath for 20 minutes. After the warming, the reaction mixture was cooled with ice, and 4 ml of an n-BuOH/pyridine mixed solution [n-BuOH:pyridine = 15:1 (volume ratio)] was added to the cooled reaction mixture. The mixture was vigorously stirred.

The reaction mixture was centrifugally separated at 2,000 rpm for 10 minutes, and the resultant supernatant was measured for an absorbance at 532 nm to determine a thiobarbituric acid reaction amount. And, on the basis of a calibration curve of mandeloaldehyde (MDA) amount prepared by using 1,1,3,3-tetramethoxypropane, a peroxidized lipid amount was determined as an amount of formed MDA.

In Control, the solution of the test compound in 10 % DMF was replaced with 0.1 ml of a 10 % DMF solution, and the above procedures were repeated to determine a peroxidized lipid amount. In Blank, each of 0.1 ml of NDAPH, 0.1 ml of ADP and 0.1 ml of $FeCl_3$ were replaced with 0.1 ml of water, the solution of the test compound in 10 % DMF was replaced with 0.1 ml of a 10 % DMF solution, and the above procedures were repeated to determine a peroxidized lipid amount.

The inhibition ratio of the lipid peroxidation reaction was calculated on the basis of the following equation.

$$\text{Inhibition ratio (\%)} =[1-(OD_1-OD_3)/(OD_2-OD_3)] \times 100$$

$OD_1$: Absorbance when test compound is added.
$OD_2$: Absorbance of Control
$OD_3$: Absorbance of Blank

Table 2 shows the results.

Table 2

| Test compound*1 | Inhibition ratio of lipid peroxidation (%)*2 | |
|---|---|---|
| | $10^{-5}$M | $10^{-6}$M |
| HQ derivative A of Reference Example 1 | 99.7 | 9.8 |
| HQ derivative B of Preparation Example 1 | -*3 | 75.9 |
| HQ derivative B of Preparation Example 2 | 100.0 | 98.9 |
| HQ derivative B of Preparation Example 3 | 100.0 | 74.8 |
| HQ derivative B of Preparation Example 4 | 99.9 | 38.3 |
| HQ derivative B of Preparation Example 5 | 63.9 | 30.8 |
| HQ derivative of Reference Example 2 | 87.2 | -*3 |
| BHT | 100.0 | 14.0 |
| HQ1 | 37.8 | -*3 |

*1: HQ stands for hydroquinone.

*2: Each of $10^{-5}$M and $10^{-6}$ indicates a concentration of test compound.

*3: Not tested.

Table 2 clearly shows that the compounds obtained in the Reference Examples have an excellent antioxidant activity.

The hydroquinone derivatives of the present invention, obtained in Preparation Examples 1 to 5, show antioxidant activity equivalent to that of BHT, a conventional typical fat-soluble antioxidant, when used in a concentration of $10^{-5}$M, and these hydroquinone derivatives also show excellent antioxidant activity over BHT when used in a low concentration as low as $10^{-6}$M.

<u>Antioxidant Activity Test Example 2</u>

[DPPH elimination ability]

The hydroquinone derivative obtained in Preparation Example 3 and BHT as test compounds were measured for the ability to eliminate DPPH (α, α-diphenyl-β-picrylhydrazyl) (active organic radical species elimination ability) in the following manner.

The test compounds were respectively dissolved in DMF to prepare DMF solutions having a test compound concentration of $10^{-2}$M.

Then, 0.02 ml each of the above DMF solutions were respectively added to 2 ml of an EtOH solution containing $10^{-4}$M of DPPH, and the resultant solutions were measured for absorbance ($OD_s$) at 517 nm at predetermined time intervals. The DPPH elimination ability of each test compound was determined on the basis of these measurement results. In Control, the DMF solution of the test compound was replaced with DMF itself.

The elimination ability of each test compound was determined as a reduction ratio of $OD_s$ to the absorbance in Control.

Figure 1 shows the results.

As is clear in Figure 1, the hydroquinone derivative B obtained in Preparation Example 3 has excellent DPPH elimination ability over BHT.

<u>Antimutagenic activity test on mutagenesis of heterocyclic amine to Salmonellae</u>

(Method)

Compounds of the present invention were tested on their antimutagenic activity to heterocyclic amines of Glu-P-1·HCl, Trp-P-1 acetate and IQ by a preincubation method-applied Ames test according to the method of Diane et al (Diane F. Birt, et al., Carcinogenesis, Vol. 7, No. 6, 959-963, 1986).

That is, S9 mix (0.5 ml), 0.1 ml of a Salmonella TA 98 medium, a compound of the present invention dissolved in DMSO and 0.1 ml of one of the above mutagens were charged into a test tube, and cultured at 37°C for 20 minutes with shaking. Then, 2 ml of soft agar was added to, and mixed with, the contents in the test tube. The resultant mixture was spread over a minimal glucose agar medium, and cultured at 37°C for 48 hours, and the number of reverse mutation colonies. Two or three plates were prepared each dosage, and the test results were compared with the test result of

a negative control group to which DMSO alone was added and the test result of a positive control group to which the corresponding mutagen alone was added. Table 3 shows the results, in which it is seen that the compounds of the present invention had the activity to inhibit the mutagenesis of heterocyclic amine.

Table 3

| Mutagen | Test compound | | Mutagenesis inhibition ratio |
|---|---|---|---|
| Glu-P-1* 0.02 μg | HQ derivative of Prep. Ex. 2 | 5.0 μg | 81 |
| | | 10.0 μg | 92 |
| Trp-P-1·acetate** 0.02 μg | ditto | 5.0 μg | 61 |
| | | 10.0 μg | 73 |
| IQ*** 0.005 μg | ditto | 5.0 μg | 89 |
| | | 10.0 μg | 64 |
| IQ*** 0.005 μg | HQ derivative of Prep. Ex. 1 | 20.0 μg | 85 |
| IQ*** 0.005 μg | HQ derivative of Ref. Ex, 2 | 20.0 μg | 85 |

*Glu-P-1·HCl: 2-amino-6-methyldipyrido[1,2-a:3',2'-d]imidazole hydrochloride
**Trp-P-1·acetate: 3-amino-1,4-dimethyl-5H-pyrido[4,3-b]indole acetate
***IQ: 2-amino-3-methylimidazo[4,5-f]quinoline

Acute toxicity test

(Method)

Female ICR mice of 8 weeks age were used to carry out an acute toxicity test by simple oral administering. Compounds of the present invention (HQ derivatives of Preparation Examples 1 to 5 and Reference Examples 1 and 2) were respectively dissolved in an olive oil, and each of the resultant solutions was orally administered to three dosage groups each consisting of 6 mice such that the maximum dose was 500 mg/kg, followed by doses gradually decreased at a common ratio of 2. The mice were observed for 14 days after the administration.

(Result)

No mice were found to die during the entire period of the observation.

As explained above, the hydroquinone derivatives of claim 1 of the present invention have antioxidant activity sufficient for use as an antioxidant. The antioxidant of the present invention contains, as an active ingredient, any one of these novel hydroquinone derivatives and/or any one of known hydroquinone derivatives defined in claim 3.

Therefore, there can be provided novel antioxidants by working the present invention, and there can be provided a wider selection of antioxidants to meet with rapidly advancing diversification in foods and cosmetics in recent years.

The hydroquinone derivatives of claim 1 of the present invention have tumor incidence inhibiting activity. The tumor incidence inhibitor of the present invention contains, as an active ingredient, any one of these novel hydroquinone derivatives and/or any one of known hydroquinone derivatives as defined in claim 7

Therefore, there can be provided novel tumor incidence inhibitors by working the present invention.

**Claims**

**1.** A hydroquinone derivative of the formula (IIa),

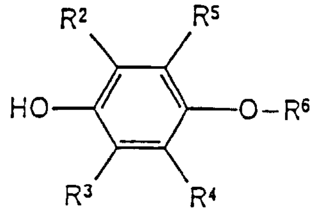

· · · (I I a)

wherein each of $R^2$ to $R^4$ is independently a $C_{1-4}$ alkyl group, $R^5$ is hydrogen and $R^6$ is an alkyl group having a carbon chain of 8 carbon atoms.

**2.** A hydroquinone derivative according to claim 1, wherein each of $R^2$ to $R^4$ is methyl.

**3.** Use of a hydroquinone derivatives as antioxidant, said hydroquinone derivative being represented by the formula (IIb),

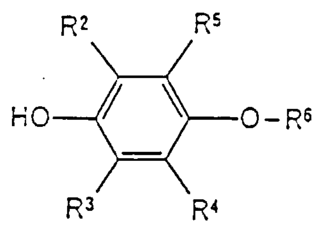

· · · (IIb)

wherein each of $R^2$ to $R^4$ is independently a $c_{1-4}$ alkyl group, $R^5$ is hydrogen and $R^6$ is an alkyl group having a carbon chain of 4 to 8 carbon atoms.

**4.** Use of a hydroquinone derivative for the preparation of a medicament for the prevention and therapy of functional disorders in organs, said hydroquinone derivative being represented by the formula (IIb),

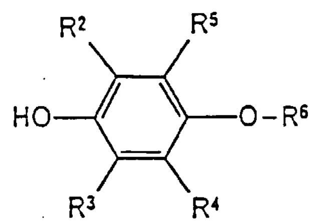

· · · (IIb)

wherein each of $R^2$ to $R^4$ is independently a $C_1$-$C_4$ alkyl group, $R^5$ is hydrogen and $R^6$ is an alkyl group having a carbon chain of 4 to 8 carbon atoms.

**5.** Use of a hydroquinone derivative for the preparation of a medicament for eliminating tissue disorder-inducing factors such as active oxygen species and active organic radical species, said hydroquinone derivative being represented by the formula (IIb),

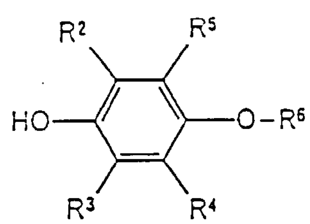

· · · (IIb)

wherein each of $R^2$ to $R^4$ is independently a $C_1$-$C_4$ alkyl group, $R^5$ is hydrogen and $R^6$ is an alkyl group having a carbon chain of 4 to 8 carbon atoms.

**6.** Use according to any of claims 3 to 5 wherein each of $R^2$ to $R^4$ is a methyl group.

**7.** Use of a hydroquinone derivative for the preparation of a medicament for the inhibition of tumor incidence, said hydroquinone derivative being represented by the formula (IIc),

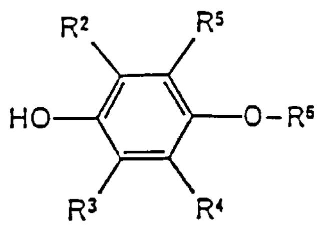

· · · (IIc)

wherein each of $R^2$ to $R^4$ is independently a $C_{1-4}$ alkyl group, $R^5$ is hydrogen and $R^6$ is an alkyl group having a carbon chain of at least 2 carbon atoms.

**8.** Use of a hydroquinone derivative according to claim 7 wherein each of $R^2$ to $R^4$ is methyl.

**Patentansprüche**

**1.** Hydrochinonderivat der Formel (IIa)

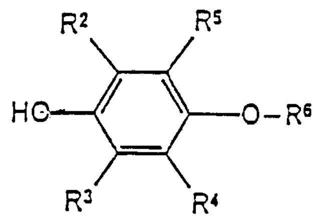

· · · (IIa)

worin $R^2$ bis $R^4$ jeweils unabhängig eine $C_{1-4}$-Alkylgruppe darstellen, $R^5$ Wasserstoff ist und $R^6$ eine Alkylgruppe mit einer Kohlenstoffkette von 8 Kohlenstoffatomen ist.

**2.** Hydrochinonderivat nach Anspruch 1, worin $R^2$ bis $R^4$ jeweils Methyl sind.

**3.** Verwendung eines Hydrochinonderivates als Antioxidationsmittel, wobei das Hydrochinonderivat durch die Formel (IIb) dargestellt wird:

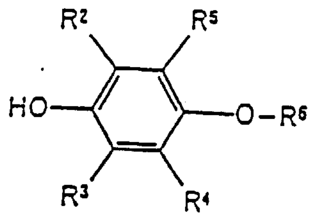

· · · (IIb)

worin $R^2$ bis $R^4$ jeweils unabhängig eine $C_{1-4}$-Alkylgruppe darstellen, $R^5$ Wasserstoff und $R^6$ eine Alkylgruppe mit einer Kohlenstoffkette von 4 bis 8 Kohlenstoffatomen ist.

**4.** Verwendung eines Hydrochinonderivates für die Herstellung eines Medikamentes für die Prävention und Therapie von funktionellen Störungen in Organen, wobei das Hydrochinonderivat durch die Formel (IIb) dargestellt wird:

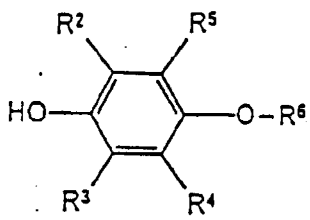

... (IIb)

worin $R^2$ bis $R^4$ jeweils unabhängig eine $C_{1-4}$-Alkylgruppe darstellen, $R^5$ Wasserstoff und $R^6$ eine Alkylgruppe ist, die eine Kohlenstoffkette von 4 bis 8 Kohlenstoffatomen aufweist.

5. Verwendung eines Hydrochinonderivates für die Herstellung eines Medikamentes zur Eliminierung von Gewebestörungeninduzierenden Faktoren wie aktiven Sauerstoffspezies und aktiven organischen Radikalspezies, wobei die Hydrochinonderivate durch die Formel (IIb) dargestellt werden:

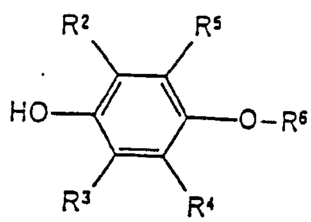

... (IIb)

worin $R^2$ bis $R^4$ jeweils unabhängig eine $C_{1-4}$-Alkylgruppe darstellen, $R^5$ Wasserstoff und $R^6$ eine Alkylgruppe ist, die eine Kohlenstoffkette von 4 bis 8 Kohlenstoffatomen aufweist.

6. Verwendung nach irgendeinem der Ansprüche 3 bis 5, worin $R^2$ bis $R^4$ jeweils eine Methylgruppe darstellt.

7. Verwendung eines Hydrochinonderivates für die Herstellung eines Medikamentes zur Inhibierung von Tumorinzidenz, wobei die genannten Hydrochinonderivate durch die Formel (IIc) dargestellt werden:

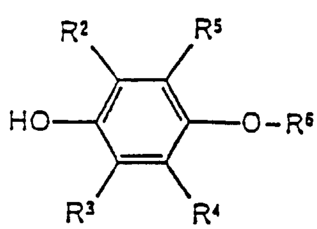

... (IIc)

worin $R^2$ bis $R^4$ jeweils unabhängig eine $C_{1-4}$-Alkylgruppe darstellen, $R^5$ Wasserstoff und $R^6$ eine Alkylgruppe ist, die eine Kohlenstoffkette von mindestens 2 Kohlenstoffatomen aufweist.

8. Verwendung eines Hydrochinonderivates nach Anspruch 7, worin $R^2$ bis $R^4$ jeweils Methyl ist.

## Revendications

1. Dérivé de l'hydroquinone de formule (IIa),

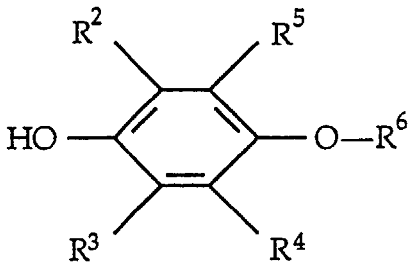

(IIa)

dans laquelle chacun des radicaux $R^2$ à $R^4$ est, indépendamment des autres, un groupe alkyle en $C_1$-$C_4$, $R^5$ est un hydrogène, et $R^6$ est un groupe alkyle ayant une chaîne carbonée constituée de 8 atomes de carbone.

2. Dérivé de l'hydroquinone selon la revendication 1, dans lequel chacun des radicaux $R^2$ à $R^4$ est le groupe méthyle.

3. Utilisation d'un dérivé de l'hydroquinone en tant qu'oxydant, ledit dérivé de l'hydroquinone étant représenté par la formule (IIb),

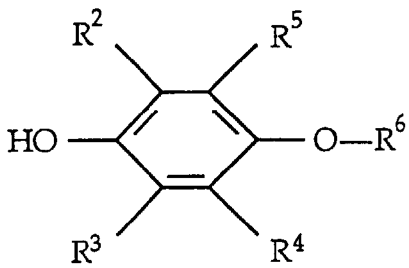

(IIb)

dans laquelle chacun des radicaux $R^2$ à $R^4$ est, indépendamment des autres, un groupe alkyle en $C_1$-$C_4$, $R^5$ est un hydrogène, et $R^6$ est un groupe alkyle ayant une chaîne carbonée constituée de 4 à 8 atomes de carbone.

4. Utilisation d'un dérivé de l'hydroquinone pour préparer un médicament destiné à la préparation et à la thérapie des troubles fonctionnels de certains organes, ledit dérivé de l'hydroquinone étant représenté par la formule (IIb),

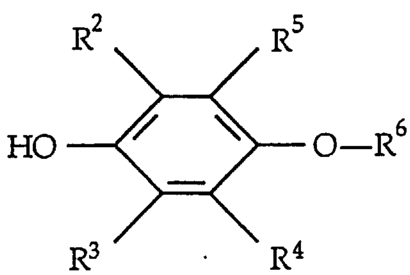

(IIb)

dans laquelle chacun des radicaux $R^2$ à $R^4$ est, indépendamment des autres, un groupe alkyle en $C_1$-$C_4$, $R^5$ est un hydrogène et $R^6$ est un groupe alkyle ayant une chaîne carbonée constituée de 4 à 8 atomes de carbone.

5. Utilisation d'un dérivé de l'hydroquinone pour préparer un médicament destiné à éliminer les facteurs provoquant des troubles tissulaires, tel qu'une espèce d'oxygène actif et une espèce de radical organique actif, ledit dérivé de l'hydroquinone étant représenté par la formule (IIb),

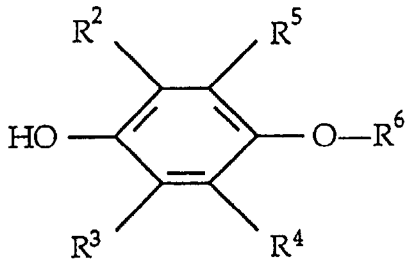

(IIb)

dans laquelle chacun des radicaux $R^2$ à $R^4$ est, indépendamment des autres, un groupe alkyle en $C_1$-$C_4$, $R^5$ est un hydrogène et $R^6$ est un groupe alkyle ayant une chaîne carbonée constituée de 4 à 8 atomes de carbone.

**6.** Utilisation selon l'une quelconque des revendications 3 à 5, dans laquelle chacun des radicaux $R^2$ à $R^4$ est un groupe méthyle.

**7.** Utilisation d'un dérivé de l'hydroquinone pour préparer un médicament destiné à inhiber l'incidence des tumeurs, ledit dérivé de l'hydroquinone étant représenté par la formule (IIc),

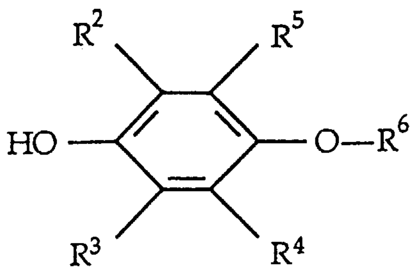

(IIc)

dans laquelle chacun des radicaux $R^2$ à $R^4$ est, indépendamment des autres, un groupe alkyle en $C_1$-$C_4$, $R^5$ est un hydrogène et $R^6$ est un groupe alkyle ayant une chaîne carbonée constituée d'au moins 2 atomes de carbone.

**8.** Utilisation d'un dérivé de l'hydroquinone selon la revendication 7, où chacun des radicaux $R^2$ à $R^4$ est le groupe méthyle.

FIG. 1